# EUROPÄISCHE PATENTSCHRIFT

(11) **EP 2 213 319 B1**
(45) Veröffentlichungstag und Bekanntmachung des Hinweises auf die Patenterteilung: **30.11.2011**
(21) Anmeldenummer: 10000708.7
(22) Anmeldetag: 25.01.2010
(51) Int. Cl.: A61M 5/20, A61M 5/142

(54) **Vorrichtung und Verfahren zur Injektion einer Flüssigkeit**
Device and method for injecting a liquid
Dispositif et procédé d'injection d'un liquide

(30) Priorität: 29.01.2009 DE 102009006680
(43) Veröffentlichungstag der Anmeldung: 04.08.2010
(73) Patentinhaber: Medtron AG, 66128 Saarbrücken (DE)
(72) Erfinder: Leder, Christoph, 66128 Saarbrücken (DE); Mehner, Gotthilf, 66280 Sulzbach (DE); Altmeyer, Hanno, 66538 Neunkirchen (DE)
(74) Vertreter: Müller-Gerbes Wagner Albiger Patentanwälte

(56) Entgegenhaltungen:
- EP-A1- 0 618 426
- EP-A1- 1 563 859
- US-A- 4 452 251
- US-A- 5 254 101

## Beschreibung

Die Erfindung betrifft eine Vorrichtung zur Injektion einer Flüssigkeit aus einer Spritze mit darin angeordnetem Spritzenkolben, umfassend eine Spritzenaufnahme zur Halterung der Spritze und eine Antriebseinrichtung für den Kolben, die mit dem Kolben der in die Spritzenaufnahme eingelegten Spritze lösbar verbindbar ist. Die Spritze ist transparent ausgeführt und weist mindestens eine Markierung auf der Spritzenwandung auf, deren optische Erscheinung sich in entleertem Zustand der Spritze von der optischen Erscheinung in mit Flüssigkeit gefülltem Zustand der Spritze unterscheidet.

Die Erfindung betrifft ferner auch ein entsprechendes Verfahren zur Injektion einer Flüssigkeit aus einer Spritze mit darin angeordnetem Spritzenkolben unter Einsatz eines Injektors, der eine Spritzenaufnahme zur Halterung der Spritze und eine Antriebseinrichtung zum Antrieb des Spritzenkolbens aufweist, wenn die Spritze in die Spritzenaufnahme eingelegt ist.

Derartige Verfahren und Vorrichtungen sind aus dem Stand der Technik bekannt, wozu lediglich beispielhaft auf die US-A-5 254 101 sowie US-A-4 452 251 verwiesen wird.

Die auf die Spritzenwandung aufgebrachte Markierung verändert aufgrund der veränderten Lichtbrechung der mit Flüssigkeit gefüllten transparenten Spritze gegenüber einer ungefüllten Spritze ihre optische Erscheinung, so dass eine visuelle Kontrolle des Füllzustandes der Spritze ermöglicht ist.

Dies ist im Einsatz mit Injektoren, wie sie beispielsweise zur Kontrastmittelinjektion im Rahmen einer angiographischen Untersuchung verwendet werden, angezeigt, da das verwendete Kontrastmittel üblicherweise ebenfalls transparent ist und von daher eine einfache visuelle Erkennung des Füllzustandes der Spritze schwierig ist. Eine solche Erkennung des Füllzustandes der Spritze ist jedoch gleichwohl dringend erforderlich, denn es besteht ansonsten die Gefahr einer Betätigung des Injektors mit einer nicht mit Flüssigkeit gefüllten Spritze, so dass dem Patienten Luft injiziert werden würde, was für den Patienten lebensbedrohlich werden kann.

Die aus dem Stand der Technik bekanntgewordenen Vorrichtungen und Verfahren verwenden eine auf die Spritzenwandung aufgebrachte Markierung, die beispielsweise kreisförmig ist und sich optisch in eine Ellipse verändert, sobald die Spritze mit Flüssigkeit gefüllt ist. Auf diese Weise wird dem Bedienungspersonal eine schnelle optische Kontrolle ermöglicht.

Nachteilig bei dem bekannten Stand der Technik ist jedoch, dass die Markierung von einer geschulten Bedienperson betrachtet werden muss, die die unterschiedlichen optischen Erscheinungsformen je nach Füllzustand der Spritze kennt und zu beurteilen vermag. Dies birgt weitere Fehlerquellen, erhöht den Schulungsaufwand und lässt die bekannten Systeme insbesondere im Rahmen von Injektoren als ungeeignet erscheinen, die vollautomatisch eine in die Spritzenaufnahme eingelegte Spritze mit Kontrastmittel befüllen, d.h. die Spritze aufziehen und nachfolgend die Injektion durchführen. In einem solchen Falle ist nämlich keine Bedienperson in der Nähe der Spritze, um den Füllzustand anhand der aufgebrachten Markierungen zu beurteilen.

Aufgabe der Erfindung ist daher, unter Umgehung der Nachteile des Standes der Technik eine Vorrichtung und ein Verfahren zur Injektion einer Flüssigkeit aus einer Spritze mittels eines Injektors vorzuschlagen, bei der zuverlässig auch im automatisierten Betrieb eine Leerinjektion, d.h. eine Injektion unter Einsatz einer nicht mit Flüssigkeit gefüllten Spritze vermieden wird.

Diese Aufgabe wird erfindungsgemäß mit einer Vorrichtung gemäß den kennzeichnenden Merkmalen des Patentanspruches 1 und einem Verfahren gemäß den kennzeichnenden Merkmalen des Patentanspruches 6 gelöst.

Vorteilhafte Ausgestaltungen und Weiterbildungen der Erfindung sind Gegenstand der jeweiligen abhängigen Ansprüche.

Erfindungsgemäß wird vorgeschlagen, dass der Spritzenaufnahme zugeordnet eine Bildauswerteeinrichtung vorgesehen ist, mittels derer bei in die Spritzenaufnahme eingelegter Spritze die Markierung der Spritze aufnehmbar und die optische Erscheinung der Markierung mit einem Referenzwert vergleichbar ist und in Abhängigkeit vom Vergleichsergebnis die Antriebseinrichtung betätigbar oder blockierbar ist.

Im Rahmen der Erfindung wird somit eine Vorrichtung zur Injektion einer Flüssigkeit aus einer Spritze mit darin angeordnetem Spritzenkolben mittels eines Injektors vorgeschlagen, die selbsttätig in der Lage ist, den Zustand der in die Spritzenaufnahme eingelegten Spritze, d.h. entweder den mit Flüssigkeit gefüllten Zustand oder auch den nicht mit Flüssigkeit gefüllten Zustand selbsttätig zu erkennen und davon abhängig die Antriebseinrichtung freizugeben bzw. zu sperren, um eine Injektion zu ermöglichen bzw. den Patienten vor einer Injektion unter Einsatz einer nicht mit Flüssigkeit gefüllten Spritze zu schützen. Die Erkennung des Spritzenzustandes wird selbsttätig mittels der Bildauswerteeinrichtung vorgenommen, ohne dass es hierfür einer Tätigkeit einer Bedienungsperson bedarf. Die Fehlersicherheit wird somit bedeutend gesteigert und es ist insbesondere möglich, einen derartigen Injektor vollständig ferngesteuert zu bedienen, einschließlich der Befüllung der eingelegten Spritze.

Bevorzugt umfasst die Bildauswerteeinrichtung eine Kamera sowie eine Steuereinrichtung zur Bildauswertung, die je nach Anwendungsfall räumlich getrennt untergebracht oder integriert angeordnet sein können.

Nach einem weiteren Vorschlag der Erfindung wird die Markierung als sich in Richtung der Längsachse der Spritze erstreckender Schriftzug ausgebildet, der auf der der Bildauswerteeinrichtung abgewandten Seite der Spritzenwandung aufgebracht ist. Der Schriftzug kann somit Informationen beispielsweise über den Hersteller der Spritze und deren Größe umfassen und dient gleichzeitig als sich mit dem Füllzustand im Erscheinungsbild ändernde Markierung, um eine mit Flüssigkeit gefüllte Spritze von einer nicht mit Flüssigkeit gefüllten Spritze einfach unterscheiden zu können.

Nach einem weiteren Vorschlag der Erfindung sind der Markierung diametral gegenüberliegend Begrenzungslinien auf die Spritzenwandung aufgebracht, deren Abstand voneinander so bemessen ist, dass bei entleerter Spritze die Markierung innerhalb der Begrenzungslinien erscheint und bei mit Flüssigkeit gefüllter Spritze aufgrund des dann geänderten Brechungsindexes die Markierung zumindest teilweise außerhalb der Begrenzungslinien erscheint. Eine solche Erscheinungsform ist mit automatisierten und im Rahmen der Erfindung vorgesehenen Bildauswerteeinrichtungen besonders leicht auswertbar, da im Rahmen der Bildauswertung lediglich entschieden werden muss, ob sich Teile der Markierung außerhalb der Begrenzungslinien befinden. Sofern dies der Fall ist, ist die gefüllte Spritze mit Sicherheit mit Flüssigkeit befüllt und die Antriebseinrichtung kann betätigt werden bzw. zur Betätigung freigegeben werden. Ermittelt die Bildauswerteeinrichtung keine Teile der Markierung außerhalb der Begrenzungslinien, so ist die in die Spritzenaufnahme eingelegte Spritze mit hoher Wahrscheinlichkeit nicht mit Flüssigkeit gefüllt und zum Schutze des Patienten vor einer ungewollten LeerInjektion wird die Antriebseinrichtung blockiert.

Darüber hinaus kann die Markierung nach einem weiteren Vorschlag der Erfindung auch eine Codierung umfassen, die ebenfalls mittels der

Bildauswerteeinrichtung lesbar ist. Diese Codierung kann beispielsweise die Spritzengröße an die Steuerung des Injektors signalisieren oder dgl. mehr.

Das erfindungsgemäße Verfahren zur Injektion einer Flüssigkeit aus einer Spritze mit darin angeordneten Spritzenkolben unter Einsatz eines Injektors beruht darauf, dass bei in die Spritzenaufnahme eingelegter Spritze die Markierung derselben mittels einer Bildauswerteeinrichtung aufgenommen und mit einem Referenzwert verglichen und in Abhängigkeit vom Ergebnis des Referenzwertvergleichs die Antriebseinrichtung aktiviert oder blockiert wird.

Erfindungsgemäß wird somit vorgeschlagen, die Erkennung der sich je nach Zustand der Spritze (mit Flüssigkeit gefüllt bzw. nicht mit Flüssigkeit gefüllt) ändernden optischen Erscheinung der Markierung mittels einer Bildauswerteeinrichtung automatisiert durchzuführen und in Abhängigkeit von der Bildauswertung die Antriebseinrichtung zu aktivieren bzw. freizugeben oder zu blockieren.

Nach einem Vorschlag der Erfindung kann die Bildauswerteeinrichtung automatisch bei injektionsbereitem Injektor aktiviert werden, was durch eine entsprechende Programmierung der ohnehin am Injektor vorhandenen Steuereinrichtung bewirkt werden kann.

Die Markierung kann entlang der Längsachse der Spritze verlaufen und im entleerten Zustand der Spritze zwischen zwei Begrenzungslinien angeordnet sein, wobei sie in mit Flüssigkeit befülltem Zustand der Spritze über die Begrenzungslinien hinaus vergrößert erscheint und von daher einfach mittels einer Bildauswerteeinrichtung evaluiert werden kann.

Die Begrenzungslinien können hierbei nach einem Vorschlag der Erfindung ebenso wie die Markierung mit geeigneten Verfahren, z.B. Bedrucken, Ätzen oder Gravieren, in die Wandung der Spritze ein- bzw. auf diese aufgebracht sein oder aber die Begrenzungslinien werden virtuell von der Bildauswerteeinrichtung mittels der darin ablaufenden Software erzeugt. Weitere Einzelheiten und Ausgestaltungen der Erfindung werden nachfolgend anhand der ein Ausführungsbeispiel darstellenden Zeichnung erläutert. Es zeigen
- Fig. 1: In schematisierter Darstellung einen Injektor gemäß der Erfindung;
- Fig. 2: Die Aufsicht auf die Spritze gemäß Figur 1;
- Fig. 3: In vergrößerter Darstellung die Markierung der Spritze in einem Zustand, in welchem die Spritze mit Flüssigkeit gefüllt ist.

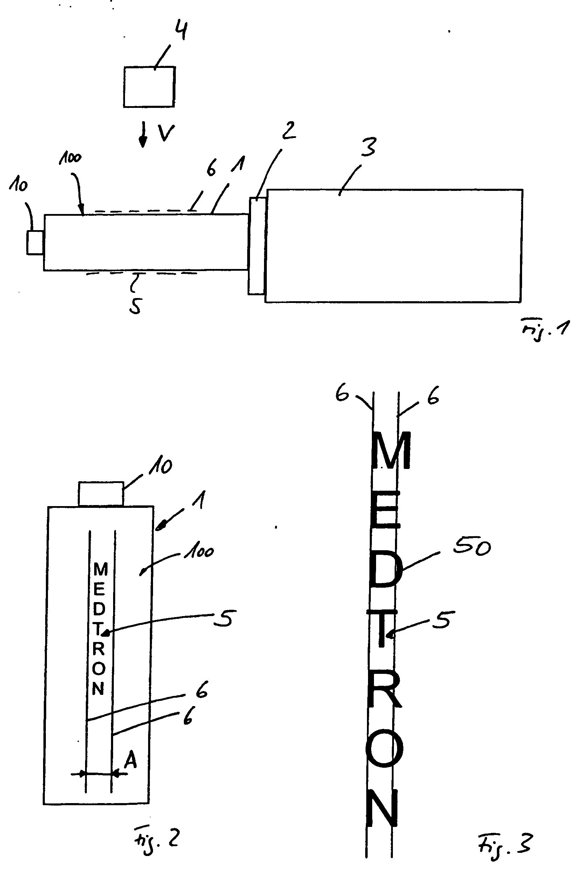

Aus der Figur 1 ist in einer stark vereinfachten und schematisierten Darstellung ein Injektor ersichtlich, der zur Injektion einer Flüssigkeit aus einer Spritze in einen nicht dargestellten Patienten dienen kann, wie es beispielsweise an bild gebenden Untersuchungseinrichtungen, wie CT oder MRT üblich ist. Bei diesen Untersuchungen ist es erforderlich, dem Patienten eine gewisse Dosis an Kontrastmittel oder dgl. zu verabreichen, was mittels des Injektors bewirkt wird, der zu diesem Zweck üblicherweise ferngesteuert ist.

Der Injektor umfasst in seinen lediglich schematisch dargestellten und wesentlichen Bauteilen eine Spritzenaufnahme 2, an bzw. in der eine mit Bezugszeichen 1 gekennzeichnete transparente Spritze 1 befestigbar ist, welche einen hier nicht näher dargestellten Spritzenkolben im Innern trägt. An das distale Ende 10 der Spritze 1 kann eine Schlauchleitung angeschlossen werden, die zum Patienten führt.

Ferner umfasst der Injektor eine mit der Spritzenaufnahme 2 verbundene Antriebseinrichtung 3, die bei in die Spritzenaufnahme 2 eingelegter Spritze 1 mit dem Kolben innerhalb der Spritze 1 verbindbar ist und den Kolben zum Aufziehen der Spritze bzw. zum Injizieren der Flüssigkeit aus der Spritze 1 betätigen kann.

Um eine ordnungsgemäße Befüllung der Spritze beispielsweise durch ein automatisiertes Aufziehen derselben mittels der Antriebseinrichtung 3 sicherzustellen und eine fehlerhafte und für den Patienten bedrohliche Injektion mittels einer nicht mit Flüssigkeit gefüllten Spritze 1 zuverlässig zu verhindern, ist der Spritzenaufnahme 2 zugeordnet oberhalb der Spritze 1 eine Bildauswerteeinrichtung 4, bestehend aus einer Kamera und einer zugeordneten Steuereinrichtung vorgesehen, die gemäß Pfeil V auf die Wandung 100 der Spritze 1 gerichtet ist.

Aufgrund einer entsprechenden Programmierung der Steuerung des Injektors wird die Bildauswerteeinrichtung 4 aktiviert, sobald der Injektor injektionsbereit ist, d.h. eine Spritze 1 in die Spritzenaufnahme 2 eingelegt und der im Innern der Spritze 1 befindliche Kolben von der Antriebseinrichtung 3 soweit vom distalen Ende 10 zurückgefahren ist, dass eine ausreichende Menge an zu injizierender Flüssigkeit im Innern der Spritze 1 vorhanden sein müsste. Ob dies tatsächlich der Fall ist, wird mittels der Bildauswerteinheit 4 ermittelt.

Aus der Figur 2 ist die Aufsicht auf die Spritze ersichtlich, wie sie sich für die in der Bildauswerteeinheit 4 integrierte Kamera unter der Blickrichtung gemäß Pfeil V darstellt. Man erkennt auf dem unteren, der Bildauswerteeinheit 4 abgewandten Bereich der Spritzenwandung 100 eine aufgebrachte Markierung 5 in Form eines sich in Längsachsenrichtung der Spritze 1 erstreckenden Schriftzuges, welcher z.B. Informationen über den Hersteller oder die Größe der verwendeten Spritze umfassen kann und z.B. aufgedruckt ist.

Auf der diametral gegenüberliegenden, d.h. der Bildauswerteeinrichtung 4 zugewandten Seite der Spritze 1 sind parallele Begrenzungslinien 6 auf die Spritzenwandung 100 aufgebracht, z.B. ebenso wie die Markierung 5 aufgedruckt.

Da die Spritze 1 insbesondere im Bereich ihrer Spritzenwandung 100 transparent ausgeführt ist, sind somit die Markierung 5 wie auch die Begrenzungslinien 6 für die Bildauswerteeinrichtung 4 sichtbar. Gegebenenfalls kann noch eine in Richtung auf die Bildauswerteeinrichtung 4 durch die Spritze 1 strahlende Lichtquelle vorgesehen sein.

Die Begrenzungslinien 6 können alternativ auch visuell in der Software der Bildauswerteeinheit 4 erzeugt werden.

Wie aus der Darstellung gemäß Figur 2 ersichtlich, ist der Abstand A zwischen den parallelen Begrenzungslinien 6 so gewählt, dass die Markierung 5 in dem Zustand der Spritze 1, in welchem diese nicht mit Flüssigkeit gefüllt ist, vollständig zwischen den beiden Begrenzungslinien 6 erscheint.

Ist hingegen die Spritze 1 mit Flüssigkeit befüllt, so ändert sich der Brechungsindex und die von der Bildauswerteeinheit 4 durch die Spritzenwandung 100 und die Flüssigkeit hindurch betrachtete Beschriftung 5 wird vergrößert dargestellt und reicht, wie aus der Figur 3 ersichtlich, zumindest bereichsweise über den von den beiden Begrenzungslinien 6 begrenzten Raum hinaus. Diese hinausreichenden Bereiche sind in der Figur 3 mit Bezugsziffer 50 beispielhaft gekennzeichnet.

Mit Hilfe einer einfachen Bildauswerteroutine ist es somit möglich, unter Zugrundelegung der beiden Begrenzungslinien 6 als Referenzwert festzustellen, ob die Markierung 5 über die Begrenzungslinien 6 hinausreicht, was den Zustand einer mit Flüssigkeit befüllten Spritze 1 signalisiert, oder aber ob die Beschriftung ausschließlich innerhalb der Begrenzungslinien 6 gemäß der darstellenden Figur 2 erscheint, was einer nicht mit Flüssigkeit gefüllten Spritze entspricht.

Im Falle der optischen Erscheinung gemäß Figur 2 wird die Antriebseinrichtung 3 blockiert, so dass es nicht zu einer Leerinjektion aus der nicht mit Flüssigkeit gefüllten Spritze 1 kommen kann, während die Antriebseinrichtung bei dem optischen Erscheinungsbild gemäß Figur 3 freigegeben wird und von daher über eine entsprechende Fernsteuerung von einer Bedienungsperson aktiviert werden kann, da Flüssigkeit im Innern der Spritze 1 vorhanden ist und eine ordnungsgemäße Injektion sichergestellt.

Die Erfindung ermöglicht von daher die automatisierte Injektion einer Flüssigkeit in einen Patienten mittels eines Injektors mit einer integrierten und ebenfalls automatisiert ablaufenden Leerspritzenerkennung.

## Patentansprüche

1. Vorrichtung zur Injektion einer Flüssigkeit aus einer Spritze (1) mit darin angeordnetem Spritzenkolben, umfassend eine Spritzenaufnahme (2) zur Halterung der Spritze (1) und einer Antriebseinrichtung (3) für den Kolben, die mit dem Kolben der in die Spritzenaufnahme (2) eingelegten Spritze lösbar verbindbar ist, wobei die Spritze (1) transparent ausgeführt ist und mindestens eine Markierung (5) auf der Spritzenwandung (100) trägt, deren optische Erscheinung sich in entleertem Zustand der Spritze (1) von der optischen Erscheinung in mit Flüssigkeit gefülltem Zustand der Spritze (1) unterscheidet, **dadurch gekennzeichnet, dass** der Spritzenaufnahme (2) zugeordnet eine Bildauswerteeinrichtung (4) vorgesehen ist, mittels derer bei in die Spritzenaufnahme (2) eingelegter Spritze (1) die Markierung (5) der Spritze (1) aufnehmbar und die optische Erscheinung der Markierung (5) mit einem Referenzwert vergleichbar ist und in Abhängigkeit vom Vergleichsergebnis die Antriebseinrichtung (3) betätigbar oder blockierbar ist.

2. Vorrichtung nach Anspruch 1, **dadurch gekennzeichnet, dass** die Bildauswerteinrichtung (4) eine Kamera sowie eine Steuereinrichtung zur Bildauswertung umfasst.

3. Vorrichtung nach Anspruch 1 oder 2, **dadurch gekennzeichnet, dass** die Markierung (5) als sich in Richtung der Längsachse der Spritze erstreckender Schriftzug ausgebildet ist, der auf der der Bildauswerteeinrichtung (4) abgewandten Seite der Spritzenwandung (100) aufgebracht ist.

4. Vorrichtung nach Anspruch 3, **dadurch gekennzeichnet, dass** der Markierung diametral gegenüberliegend Begrenzungslinien (6) auf die Spritzenwandung (100) aufgebracht sind, deren Abstand A voneinander so bemessen ist, dass bei entleerter Spritze die Markierung (5) innerhalb der Begrenzungslinien (6) erscheint und bei mit Flüssigkeit gefüllter Spritze (1) die Markierung (5) zumindest teilweise außerhalb der Begrenzungslinien (6) erscheint.

5. Vorrichtung nach einem der Ansprüche 1 bis 4, **dadurch gekennzeichnet, dass** die Markierung (5) eine Codierung umfasst, die mittels der Bildauswerteeinrichtung (4) auslesbar ist.

6. Verfahren zur Injektion einer Flüssigkeit aus einer Spritze (1) mit darin angeordnetem Spritzenkolben unter Einsatz eines Injektors, der eine Spritzenaufnahme (2) zur Halterung der Spritze und eine Antriebseinrichtung (3) zum Antrieb des Spritzenkolbens aufweist, wenn die Spritze (1) in die Spritzenaufnahme (2) eingelegt ist, wobei die Spritze (1) transparent ausgeführt ist und mindestens eine Markierung (5) auf der Spritzenwandung (100) trägt, deren optische Erscheinung sich in entleertem Zustand der Spritze (1) von der optischen Erscheinung in mit Flüssigkeit gefülltem Zustand der Spritze (1) unterscheidet, **dadurch gekennzeichnet, dass** bei in die Spritzenaufnahme (2) eingelegter Spritze (1) die Markierung (5) derselben mittels einer Bildauswerteeinrichtung (4) aufgenommen und mit einem Referenzwert verglichen wird und in Abhängigkeit vom Ergebnis des Referenzwertvergleichs die Antriebseinrichtung (3) aktiviert oder blockiert wird.

7. Verfahren nach Anspruch 6, **dadurch gekennzeichnet, dass** die Bildauswerteeinrichtung (4) bei injektionsbereitem Injektor aktiviert wird.

8. Verfahren nach Anspruch 6 oder 7, **dadurch gekennzeichnet, dass** die Markierung (5) entlang der Längsachse der Spritze (1) verläuft und in entleertem Zustand der Spritze zwischen zwei Begrenzungslinien (6) angeordnet ist und in mit Flüssigkeit befüllten Zustand der Spritze (1) über die Begrenzungslinien (6) hinaus vergrößert erscheint.

9. Verfahren nach Anspruch 8, **dadurch gekennzeichnet, dass** die Begrenzungslinien (6) auf die Spritzenwandung (100) aufgebracht sind oder virtuell von der Bildauswerteeinrichtung erzeugt werden.

10. Verfahren nach einem der Ansprüche 6 bis 9, **dadurch gekennzeichnet, dass** die Markierung (5) eine Codierung umfasst, die von der Bildauswerteeinrichtung (4) ausgelesen und an eine Steuerung des Injektors weitergeleitet wird.

## Claims

1. Device for injecting a liquid from a syringe (1) with a syringe plunger arranged therein, comprising a syringe holder (2) for holding the syringe (1) and a plunger drive mechanism (3), which can be releasably connected to the plunger of the syringe inserted in the syringe holder (2), the syringe (1) being transparent and carrying at least one marking (5) on the syringe wall (100), the visual appearance of which, in the emptied state of the syringe (1), differs from the visual appearance in the liquid-filled state of the syringe (1), **characterised in that** an image evaluation device (4) is provided, associated with the syringe holder (2), by means of which image evaluation mechanism, when the syringe (1) is inserted in the syringe holder (2), the marking (5) of the syringe (1) can be recorded and the visual appearance of the marking (5) can be compared with a reference value and, depending on the comparison result, the drive mechanism (3) can be actuated or blocked.

2. Device according to claim 1, **characterised in that** the image evaluation device (4) comprises a camera and a control device to evaluate the image.

3. Device according to claim 1 or 2, **characterised in that** the marking (5) is configured as writing extending in the direction of the longitudinal axis of the syringe, the writing being applied to the side of the syringe wall (100) remote from the image evaluation device (4).

4. Device according to claim 3, **characterised in that** limit lines (6) are applied to the syringe wall (100) diametrically opposing the marking, the spacing A of which from one another is such that when the syringe has been emptied, the marking (5) appears within the limit lines (6) and when the syringe (1) is filled with liquid, the marking (5) appears at least partially outside the limit lines (6).

5. Device according to any one of claims 1 to 4, **characterised in that** the marking (5) comprises a coding, which can be read by means of the image evaluation device (4).

6. Method for injecting a liquid from a syringe (1) with a syringe plunger arranged therein using an injector, which has a syringe holder (2) for holding the syringe and a drive mechanism (3) to drive the syringe plunger when the syringe (1) is inserted in the syringe holder (2), the syringe (1) being transparent and carrying at least one marking (5) on the syringe wall (100), the visual appearance of which, in the emptied state of the syringe (1), differs from the visual appearance in the liquid-filled state of the syringe (1), **characterised in that** when the syringe (1) is inserted in the syringe holder (2), the marking (5) thereof is recorded by means of an image evaluation device (4) and compared with a reference value and the drive mechanism (3) is activated or blocked depending on the result of the reference value comparison.

7. Method according to claim 6, **characterised in that** the image evaluation device (4) is activated, when the injector is ready for injection.

8. Method according to claim 6, or 7, **characterised in that** the marking (5) runs along the longitudinal axis of the syringe (1) and, in the emptied state of the syringe, is arranged between two limit lines (6) and, in the liquid-filled state of the syringe (1), appears to be enlarged beyond the limit lines (6).

9. Method according to claim 8, **characterised in that** the limit lines (6) are applied to the syringe wall (100) or are produced virtually by the image evaluation device.

10. Method according to any one of claims 6 to 9, **characterised in that** the marking (5) comprises a coding, which is read by the image evaluation device (4) and passed to a controller of the injector.

## Revendications

1. Dispositif d'injection d'un liquide depuis une seringue (1) contenant un piston d'injection, comprenant un logement de seringue (2) pour la fixation de la seringue (1) et un dispositif d'entraînement (3) pour le piston qui peut être relié de manière amovible au piston de la seringue insérée dans le logement de seringue (2), sachant que la seringue (1) est réalisée transparente et porte au moins un marquage (5) sur sa paroi (100), dont l'apparence optique se distingue à l'état vidé de la seringue (1) de l'apparence optique à l'état rempli de liquide de la seringue (1), **caractérisé en ce qu'**un dispositif d'évaluation d'image (4) associé au logement de seringue (2) est prévu, à l'aide duquel lorsque la seringue (1) est insérée dans le logement d'aiguille (2), le marquage (5) de la seringue (1) peut être enregistré et l'apparence optique du marquage (5) peut être comparée à une valeur de référence et le dispositif d'entraînement peut être actionné ou bloqué en fonction du résultat de comparaison.

2. Dispositif selon la revendication 1, **caractérisé en ce que** le dispositif d'évaluation d'image (4) comporte une caméra ainsi qu'un dispositif de commande pour l'évaluation d'image.

3. Dispositif selon la revendication 1 ou 2, **caractérisé en ce que** le marquage (5) est réalisé comme une écriture s'étendant en direction de l'axe longitudinal de la seringue et appliquée sur le côté éloigné du dispositif d'évaluation d'image (4) de la paroi de seringue (100).

4. Dispositif selon la revendication 3, **caractérisé en ce que** des lignes de délimitation (6) sont appliquées de manière diamétralement opposée au marquage sur la paroi de seringue (100), dont la distance A les unes par rapport aux autres est mesurée de sorte qu'en cas de seringue vidée, le marquage (5) apparaisse dans les lignes de délimitation (6) et en cas de seringue (1) remplie de liquide, le marquage (5) apparaisse au moins en partie en dehors des lignes de délimitation (6).

5. Dispositif selon l'une quelconque des revendications 1 à 4, **caractérisé en ce que** le marquage (5) comporte un codage qui peut être lu à l'aide du dispositif d'évaluation d'image (4).

6. Procédé d'injection d'un liquide depuis une seringue (1) contenant un piston d'injection, en utilisant un injecteur qui présente un logement de seringue (2) pour la fixation de la seringue et un dispositif d'entraînement (3) pour l'entraînement du piston d'injection, lorsque la seringue (1) est insérée dans le logement de seringue (2), sachant que la seringue (1) est réalisée transparente et porte au moins un marquage (5) sur sa paroi (100), dont l'apparence optique se distingue à l'état vidé de la seringue (1) de l'apparence optique à l'état rempli de liquide de la seringue (1), **caractérisé en ce que** lorsque la seringue (1) est insérée dans le logement de seringue (2), le marquage (5) est enregistré à l'aide d'un dispositif d'évaluation d'image (4) et est comparé à une valeur de référence et le dispositif d'entraînement (3) est activé ou bloqué en fonction du résultat de la comparaison avec la valeur de référence.

7. Procédé selon la revendication 6, **caractérisé en ce que** le dispositif d'évaluation d'image (4) est activé en cas d'injecteur prêt à l'injection.

8. Procédé selon la revendication 6 ou 7, **caractérisé en ce que** le marquage (5) s'étend le long de l'axe longitudinal de la seringue (1) et est disposé à l'état vidé de la seringue entre deux lignes de délimitation (6) et apparaît agrandi à l'état rempli de liquide de la seringue (1) au-delà des lignes de délimitation (6).

9. Procédé selon la revendication 8, **caractérisé en ce que** les lignes de délimitation (6) sont appliquées sur la paroi de seringue (100) ou sont générées virtuellement par le dispositif d'évaluation d'image.

10. Procédé selon l'une quelconque des revendications 6 à 9, **caractérisé en ce que** le marquage (5) comporte un codage qui est lu par le dispositif d'évaluation d'image (4) et est transmis à une commande de l'injecteur.
